# EUROPEAN PATENT APPLICATION

(11) **EP 1 406 202 A2**
(43) Date of publication of application: **07.04.2004**
(21) Application number: 03255577.3
(22) Date of filing: 08.09.2003
(51) Int. Cl.: G06F 19/00

(54) **Medical image management apparatus and system**

(30) Priority: 12.09.2002 JP 2002266221
(71) Applicant: KONICA CORPORATION, Tokyo 163-0512 (JP)
(72) Inventor: Onishi, Tetsuya, Hachioji-shi Tokyo 192-8505 (JP)
(74) Representative: Rees, Alexander Ellison

(57) **Abstract**

An image management apparatus capable of receiving a medical image from a medical image generating apparatus according to a radiographing order issued by an outer information management apparatus, image-processing the medical image received, displaying the medical image image-processed along with accompanying information thereof and outputting the medical image and the accompanying information to at least one of an image forming apparatus and an image storage apparatus. The image management apparatus has: a request section for requesting at least one of the medical image generating apparatus and the outer information management apparatus to have the medical image generating apparatus at least one of re-generate and resend the medical image, in a case of being impossible to apply image processing suitable for at least one of image-forming in the image forming apparatus and image-storing in the image storage apparatus on the medical image received.

## Description

### BACKGROUND OF THE INVENTION

### Field of the Invention

The invention relates to an image management apparatus and a medical network system capable of receiving a medical image from a medical image generating apparatus, image-processing the received medical image, displaying the image-processed medical image and accompanying information, and outputting them to at least one of an image forming apparatus and an image storage apparatus.

### Description of Related Art

Conventionally, as disclosed in Japanese Translation Publication of PCT International Application No. Tokuhyo-Hei 6-78910 for example, an image management apparatus managing medical images such as radiographic images or the like receives the medical images such as radiographic images or the like, and displays the received medical images. Further, the image management apparatus processes and converts the medical images into images suitable for image-storing and image-forming to be outputted to at least one of an image storage apparatus and an image forming apparatus that are located externally.

When it is not possible to store the medical image in the image storage apparatus or to form the medical image in the image forming apparatus because of any one of a case that quality of the medical image the image management apparatus has received is bad, and a case that the received medical image is wrong, it is necessary to abandon the medical image and travel to an HIS/RIS to re-issue a radiographing order. Further, in a case that the quality of the received medical image or accompanying information regarding the received medical image is not sufficient, it is also necessary to redo the image processing at the image management apparatus or to re-issue a radiographing order.

### SUMMARY OF THE INVENTION

An object of the present invention is to provide an image management apparatus and a medical network system capable of receiving image information which has been adjusted or corrected by a medical image generating apparatus in any one of a case that quality of a medical image received from a medical image generating apparatus is bad, and a case that accompanying information of the received medical image is not sufficient.

In accordance with a first aspect of the present invention, an image management apparatus capable of receiving a medical image from a medical image generating apparatus according to a radiographing order issued by an outer information management apparatus, image-processing the medical image received, displaying the medical image image-processed along with accompanying information thereof and outputting the medical image and the accompanying information to at least one of an image forming apparatus and an image storage apparatus, comprises: a request section for requesting at least one of the medical image generating apparatus and the outer information management apparatus to have the medical image generating apparatus at least one of re-generate and re-send the medical image, in a case of being impossible to apply image processing suitable for at least one of image-forming in the image forming apparatus and image-storing in the image storage apparatus on the medical image received.

In accordance with a second aspect of the present invention, a medical network system comprises: an image management apparatus, a medical image generating apparatus for sending a medical image to the image management apparatus, at least one of an image storage apparatus for storing the medical image outputted from the image management apparatus and an image forming apparatus for forming the medical image outputted in a recording medium therein, and an information management apparatus for issuing a radiographing order to the medical image generating apparatus and managing information, wherein the image management apparatus comprises a request section for requesting at least one of the medical image generating apparatus and the information management apparatus to have the medical image generating apparatus at least one of re-generate and re-send the medical image, in a case of being impossible to apply image processing suitable for at least one of image-forming in the image forming apparatus and image-storing in the image storage apparatus on the medical image received.

According to the apparatus of the first aspect of the present invention or the system of the second aspect of the present invention, for example, in a case that a medical image suitable for image-forming or image-storing cannot be obtained or the like, since it is possible to request to have the medical image generating apparatus re-generate and re-send the medical image automatically, it is possible to receive image information such as the medical image re-generated with correction or adjustment by the medical image generating apparatus, accompanying information thereof or the like automatically. Consequently, it is possible that the image management apparatus obtains the image information having a quality medical image and sufficient accompanying information. Further, the image management apparatus can receive the re-generated image information automatically without having a user travel to the outer information management apparatus.

Preferably, in the apparatus of the first aspect of the present invention or the system of the second aspect of the present invention, the request section sends additional information so as to have the medical image generating apparatus re-generate the medical image when requesting at least one of the medical image generating apparatus and the outer information management apparatus to have the medical image generating apparatus re-generate the medical image.

More preferably, in the above-mentioned apparatus or the above-mentioned system, the additional information so as to have the medical image generating apparatus re-generate the medical image includes information for instructing the medical image generating apparatus to send any one of the medical image which already has been processed, and a combination of the medical image which has not been processed and contents of the image processing performed by the medical image generating apparatus.

Preferably, in the apparatus of the first aspect of the present invention or the system of the second aspect of the present invention, the case of being impossible to apply image processing includes at least one of a case that a medical image image-processed by at least one of the medical image generating apparatus, the image management apparatus and the image forming apparatus is not optimum and a case that the accompanying information is insufficient.

Preferably, in the apparatus of the first aspect of the present invention or the system of the second aspect of the present invention, a medical standard network protocol is used for communication for requesting to have the medical image generating apparatus re-generate the medical image.

### BRIEF DESCRIPTION OF THE DRAWINGS

The present invention will become more fully understood from the detailed description given hereinafter and the accompanying drawing given by way of illustration only, and thus are not intended as a definition of the limits of the present invention, and wherein:
FIG. 1 is a view showing a structure of a medical network system according to an embodiment of the present invention,
FIG. 2 is a block diagram showing a structure of an image management apparatus 50 shown in FIG. 1,
FIG. 3 is a block diagram showing a control system for controlling the image management apparatus 50 shown in FIG. 1 and FIG. 2 in order to receive images adjusted or corrected by a medical image generating apparatus 51, 56, 57 or 59, and
FIG. 4A and FIG. 4B are flow charts for explaining each step of operation of the image management apparatus 50 shown in FIG. 1 and FIG. 2.

### EMBODIMENTS OF THE INVENTION

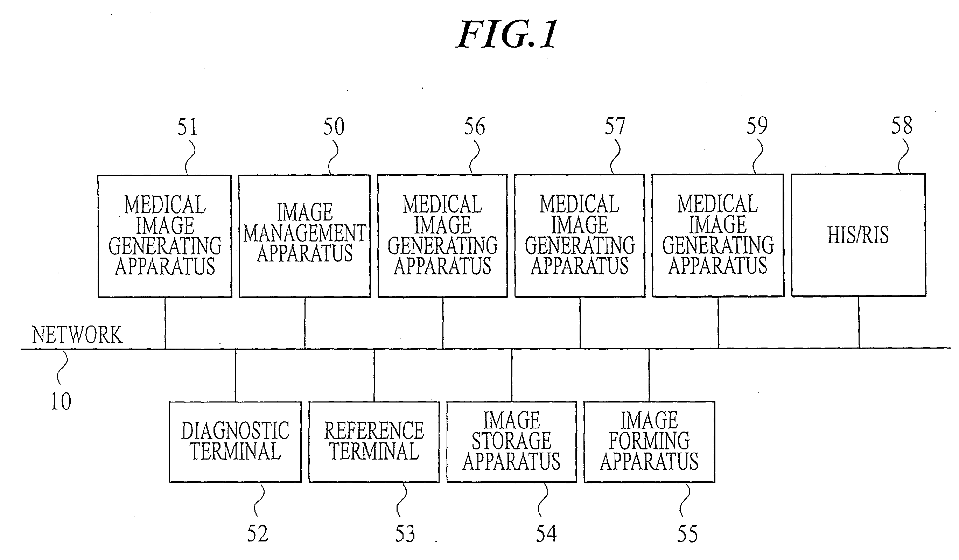

Hereinafter, a medical network system of an embodiment of the present invention will be explained with reference to figures. FIG. 1 is a view showing a structure of the medical network system according to the embodiment of the present invention.

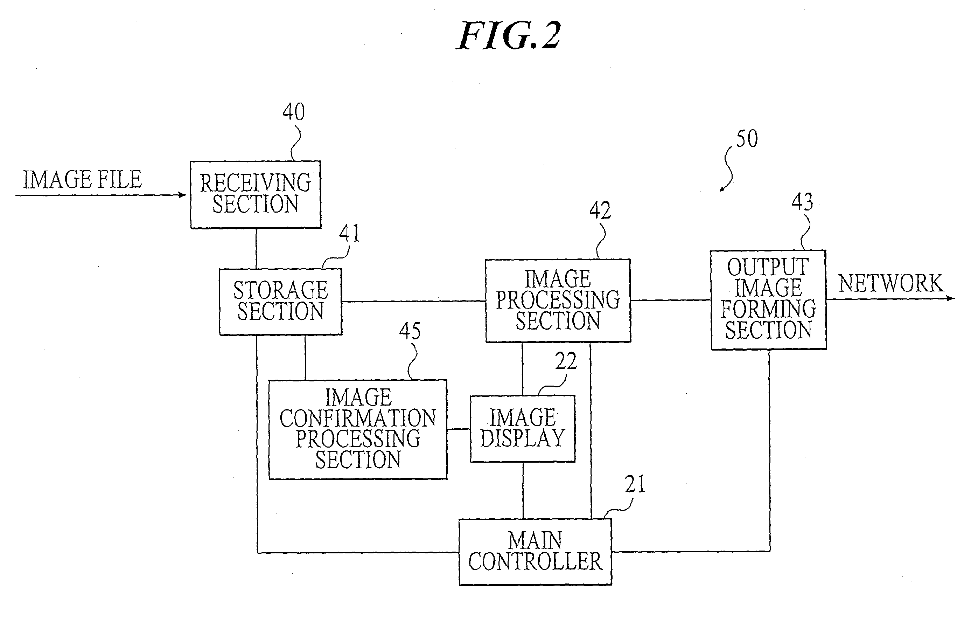

An image management apparatus 50 shown in FIG. 1 receives medical images so as to apply image processing on them and either outputs the image-processed medical images to an outer apparatus or displays the image-processed medical images on a screen of an image display 22 (FIG. 2), the medical images generated by scanning a photostimulable phosphor panel accumulating radiation image information of a subject (a patient) with excitation light for emitting stimulated light and photoelectrically converting the stimulated light with a photomultiplier, or the medical images generated by a modality such as a CR (Computed Radiography) or the like obtaining image information with an X-ray flat panel detector.

As shown in FIG. 1, in addition to the image management apparatus 50, medical image generating apparatuses 51 and 56 for generating medical images with the CR as mentioned above, medical image generating apparatuses 57 and 59 for generating medical images with a CT (Computed Tomography), a diagnostic terminal 52 in order for a roentgenolotgist to refer to images for diagnoses, a reference terminal 53 for referring to images (not for diagnoses), an image storage apparatus 54 capable of storing image files in an image database, and searching and reading an image from the diagnostic terminal 52, the reference terminal 53 or the like, and an image forming apparatus (an imager, a printer) 55 for outputting image information from either the medical image generating apparatus 51 or the image management apparatus 50 onto a recording medium such as film, paper or the like as a visible image, constitute the medical network system.

In the medical network system in FIG. 1, each apparatus is connected on-line through a network 10 for sending and receiving information to each other. Further, an HIS (Hospital Information System) / RIS (Radiology Information System) 58 for issuing a radiographing order is connected to the network 10 so that the image management apparatus 50 can communicate with the HIS/RIS 58 with a medical standard network protocol (for example, DICOM, HL7 or the like) and request the HIS/RIS 58 to have any one of the medical image generating apparatuses 51, 56, 57 and 59 re-generate the medical image or the like.

Further, in medical image generating apparatuses 51, 56, 57, 59, the image storage apparatus 54 and the image forming apparatus 55, it is possible to apply predetermined image processing on each of received medical images by user operation.

Next, the following items A - H relating to the image management apparatus 50 in FIG. 1 will be explained in detail successively.
A. Apparatus structure
B. Information
C. File
D. Input and display of main information
E. Image confirming procedures
F. Output
G. Output image formation
H. Utility function

A. Apparatus structure

FIG. 2 is a block diagram showing an internal structure of the image management apparatus 50.
a. As shown in FIG. 2, the image management apparatus 50 comprises a main controller 21 for controlling operation of the whole apparatus, a receiving section 40 for receiving image files from the medical image generating apparatus 51 or the like, a storage section 41 that is composed of a hard disk, a RAM or the like and stores information such as the received image files or the like, an image processing section 42 for conducting image processing on image information in the image files, an output image forming section 43 for forming output images to be outputted to outer apparatuses, an image confirmation processing section 45 for making a image display 22 display reduced images for confirmation or the like of the received images thereon, and the image display 22 that is composed of a CRT display, a liquid crystal panel or the like and displays images and various types of image data such as accompanying information thereof or the like.
   The main controller 21 controls each of sections 40, 41, 42, 43, 45, the image display 22 and the like. It is possible to input various types of information to the main controller 21 or the like from an operation section 21b such as an input keyboard, a mouse or the like provided at the image management apparatus 50.
b. Functions of the image management apparatus 50 are as follows. Each function is controlled by the main controller 21.
   (1) To receive image files generated by the medical image generating apparatus 51 or the like at the receiving section 40.
   (2) To store image files in the storage section 41 temporarily.
   (3) To confirm image quality by use of reduced images prepared by the image confirmation processing section 45.
   (4) To conduct image processing at the image processing section 42.
   (5) To form output images at the output image forming section 43.
   (6) To transmit output images to outer apparatuses such as the image storage apparatus 54 or the like through the network 10.

### B. Information

Information handled by the image management apparatus 50 can be classified as follows.

### a. Condition information

The condition information is necessary for receiving image files and for outputting the received image files to outer apparatuses such as the image storage apparatus 54 or the like as processed image files. The condition information includes the following:

### (a) Image processing information

The image processing information concerns gradation processing or frequency processing at the image processing section 42.

### (b) Output apparatus information

The output apparatus information concerns outer apparatuses such as the image storage apparatus 54 or the like that reproduce and output image data. It specifies an output area, an enlargement/reduction ratio, output format (multi-format, split radiographing format), overlay, whether to conduct gradation processing and frequency processing or the like.

### (c) Overlay information

The overlay information concerns whether to overlay AP/PA, R/L, comments or the like, a position of the overlay or the like.

### (d) Information about specific designation

Information of protection: To store image files even after the image files are transmitted until protection is removed.

Information of postponement: To postpone the transmission. The information of postponement is specified when it is necessary to review and transmit the image later.

Information of priority (emergency): The information of priority is specified when preferential output such as emergency radiographing is required. In this case, the radiographing is registered at the top of a queue.

### b. Patient information

The patient information concerns a patient.

### (a) Patient ID information

The patient ID information includes an ID number, a full name, a sex, a date of birth of a patient or the like.

### (b) Order information

The order information is necessary for a doctor to request radiographing. The order information includes information concerning patient conditions, instructions of a date and a method for the requested examination or the like.

### c. Implementation information

The implementation information concerns a result of receiving and image processing.
(a) The implementation information includes a result of receiving, a date of radiographing or the like.
(b) A result of image processing
   The result of image processing is a result of calculation of an image processing parameter. Image data is processed based on the result when being outputted.
(c) Implementation system information

The implementation system information includes a part of system information as follows such as a system structure at the time when radiographing is conducted or the like.

### d. System information

(a) Information for managing and controlling the system in FIG. 1.
(b) A structure of the system in FIG. 1 (identification information of an outer apparatus connected thereto such as the image storage apparatus 54 or the like, a name of the apparatus or the like).
(c) A parameter or a table for controlling devices constituting the system in FIG. 1.
(d) Setting information concerning the medical image generating apparatuses 51, 56, 57 and 59 that are inputting apparatuses.
(e) Setting information concerning an output apparatus such as information of the image forming apparatus 55, HOST information or the like.

### e. Image data

(a) Image data received from the medical image generating apparatus 51.
(b) Reduced image data for display prepared from image data for image confirmation.
(c) Reduced image data for image processing of the reduced image for display at the image confirmation processing section 45.
(d) Output image data on which gradation processing, frequency processing or the like have been applied.

### C. File

A file handled by the image management apparatus 50 is stored in the storage section 41, and is classified as follows.

### a. Condition file

A condition key is a key for setting image process conditions and output conditions on image files in advance, and a condition file corresponding to each condition key is prepared. The condition file is composed of the above-mentioned condition information. The condition file is classified in terms of radiographic regions (lung, abdomen, head or the like), radiographing postures (erect posture, supine posture or the like), radiographing directions (front, side or the like), characteristics of a patient (sex, age, physique or the like), a disease name, a radiologist or the like, and a name and radiographing information corresponding to each of the above-mentioned classifying factors are set in advance. Then, the main controller 21 sets a condition file group corresponding to each of the plurality of classifications, then sets a plurality of condition files for every set condition file group, and stores the plurality of condition files in the storage section 41. Then, the optimum condition is selected at the time of receiving images.

### b. Image header file

After the image is received, an image header file is prepared. The image header file is composed of a reservation file of the radiographing (namely, radiographing information and patient information) and implementation information. When a user refers to the photographing information, the patient information or the implementation information for changing, the user refers to the image header file.

### c. Reduced image file

The reduced image file is obtained by reducing image data at a certain reduction ratio.

### (a) Reduced image data for display

The reduced image data for display is used as data displayed on the image display 22 in FIG. 2.

### (b) Reduced image data for image processing

The reduced image data for image processing is used for calculating a parameter in order to conduct image processing. Its reduction ratio is determined by conforming length of one pixel after the reduction to predetermined length. As a result, after the reduction, it is possible to compensate the difference of reading pixel sizes. The calculation of a parameter for image processing is not conducted on original image data but conducted on the reduced image data for image processing.

### d. Image file

(a) An image file is composed of accompanying information (an image header) and image data.
(b) An image header is composed of the condition information, the patient information and the implementation information. When a user needs to refer to any one of the condition information, the patient information and the implementation information for changing, the user refers to the image header.

### e. Output image file

The output image file is a file of output image data on which predetermined processing among frequency processing, gradation processing, overlay processing, rotation processing and enlargement/reduction processing has been applied.

### f. System file

The system file is a file of the above-mentioned system information.

### D. Input and display of primary information

### a. Received image information display

Received images are displayed in thumbnail mode.

### b. Output information display

(1) An output size, a direction, a trimming position, an output position, an enlargement/reduction method or the like is determined and registered in the condition file in advance.
(2) When the condition key is selected, an output range and an output image range are determined under the predetermined condition and displayed on a screen of the image display 22. Here, a size of an output range display area on the screen of the image display 22 is the maximum output range at output. The output range and the output image range are graphically displayed on the output range display area so that an appropriate output range and an appropriate output image range can be selected and confirmed at each apparatus.

### c. Overlay information

(1) The overlay information specifies whether to overlay "AP", "PA", "R", "L", comments, calibration or the like, and a position of the overlay. The specification is registered in the condition file in advance.
(2) When output images are displayed within the output range display area on the screen of the image display 22, the overlay information is graphically displayed therewith.
(3) It is possible to select an appropriate overlay and specify its position.
(4) It is possible to confirm whether there is no invisible portion hidden behind the overlay. When the overlay causes inconvenience for diagnoses, the overlay can be moved.

### d. Input and output of on-line information from RIS

(1) When orders from a doctor are inputted, the inputted orders are converted into a data structure applicable to the system to be stored in the reservation file. Further, the radiographic region and the radiographing method are converted into corresponding radiographing condition keys.
(2) The image header file is converted into a data structure applicable to the RIS side to be outputted.

### e. Image list

Image files can be displayed as a list

### E. Image confirmation procedures

### a. Operation of the system at image confirmation

(1) When image files are received from the medical image generating apparatus 51, the image files are stored in the storage section 41.
(2) The image files stored in a storage medium of the storage section 41 are reduced at a predetermined reduction ratio by the image confirmation processing section 45.
(3) The reduced images are successively displayed on the screen of the image display 22.
(4) After all the reduced images are received and displayed, digital image information is image-processed with a method predetermined by the condition key to be displayed on the image display 22. The reduced images are used to determine a parameter for the image processing.
(5) When an operator observes received images displayed on the image display 22 and judges that they are normal, a key for confirming the completion of receiving is inputted from a character information inputting apparatus so as to terminate the image confirmation.
(6) When any one of the patient information, the image processing method, an output method and so on needs to be changed, it is possible to input new information from the character information inputting apparatus.
(7) When an image confirming key is inputted, the confirmation of the image is terminated and a following image is displayed automatically.
(8) When an image has a problem, it is possible to change image processing. Further, it is possible to postpone the confirmation of the image and change the detail of image processing later.
(9) When the image confirming key is inputted, the confirmation of the image is terminated and processing as follows is conducted:
   (9-1) The image file is stored in the storage section 41 as a confirmed image file.
   (9-2) Images of which confirmation has been terminated are registered in a queue for outputting to an outer apparatus. (9-3) Then, the following received image file is displayed for image confirmation.
(10) When a postponement key is inputted, image confirmation is terminated.

### F. Output

(1) Output is conducted on a non-synchronization basis with the receiving or the image confirmation.
(2) A queue is prepared and managed at each outer apparatus. The queues are operated independently, and therefore they have no influence to each other. As a result, output is done at each apparatus on a non-synchronization basis.
(3) Information on the outer apparatus having the queue in which the image is registered, is stored in the storage section 41 as a queue registration table. The queue registration table is updated and controlled for each registration and cancellation.
(4) The image registered in the queue is outputted to an outer apparatus in the order of registration thereof. The image which has been outputted is deleted from the queue.
(5) When the output is carried out, an image file stored in the storage section 41 is identified from the number registered in the queue.
(6) An output image is formed under the condition stored in the image file. The image header is converted into a data structure determined at each output apparatus, and transmitted along with image data.

### G. Forming output image

a. An output image is formed by the output image forming section 43 through the following processing:
   (1) Image data is read out of the storage section 41 to a memory for images.
   (2) Frequency processing is conducted.
   (3) Equalization processing is conducted.
   (4) Gradation processing is conducted.
   (5) Rotation of an image is carried out.
   (6) Mirror reversing is conducted.
   (7) Enlargement and reduction are carried out.
   (8) Overlay is carried out.
b. With respect to each of (2) - (8), whether it is executed or not can be specified at each output apparatus according to condition information.
c. Here, it is possible to specify that image data on which each of the specified processing of (2) - (8) has been applied is stored as a processed image data file. As a result, re-processing of common processing on the output image to each output apparatus can be avoided.
d. For example, when an enlargement ratio or a reduction ratio of an output image for an output apparatus is different depending on each output apparatus, images on which processing up to (6) has been applied may be stored. As a result, it is possible to shorten a period of time for (2) - (6) by reading the stored images to apply only (7) and (8), when the images are transmitted to another apparatus.
e. Further, processing of (5) and (6) may be conducted simultaneously with any one of (2), (3) and (4). As a result, it is possible to reduce access of memory and shorten processing time.

### H. Utility function

a. To provide a user several functions as a utility. Utility function is restricted by a password for each of a general user, a manager and a maker, respectively. In particular, for a change of information relating to images, a password of the manager is required in view of security.
b. Image file operation
   (1) An image file list, which is information concerning images stored, is displayed on the image display 22 in the order of receiving.
   (2) When a desired image is selected from the list, the patient information, the condition information and images are displayed in the same form as the screen at the image confirmation.
   (3) The patient information, the image processing method and the output method and so on can be changed.
   (4) With regard to the image specified as "postponement" at the time of radiographing, the "postponement" is removed at this point by reviewing the image.
   (5) The order of output, and whether output to each outer apparatus is conducted can be changed.
c. Radiographing record, emission record
   (1) The radiographing information and the patient information are processed statistically for providing a user as a radiographing record and an emission record.
   (2) The number of shots on each radiographic region for a predetermined period, a list of radiographing conditions used to radiograph for a day or the like can be outputted.
d. Customizing

A screen and operating procedures can be customized to each user.

Next, operation and a structure of the image management apparatus 50 shown in FIG. 1 and FIG. 2 will be explained with reference to figures, the operation and the structure capable of receiving the image information adjusted or corrected by any one of the medical image generating apparatuses 51, 56, 57 and 59, in a case that quality of a medical image received from any one of the medical image generating apparatuses 51, 56, 57 and 59 is bad or a case that accompanying information regarding the received medical image is not sufficient.

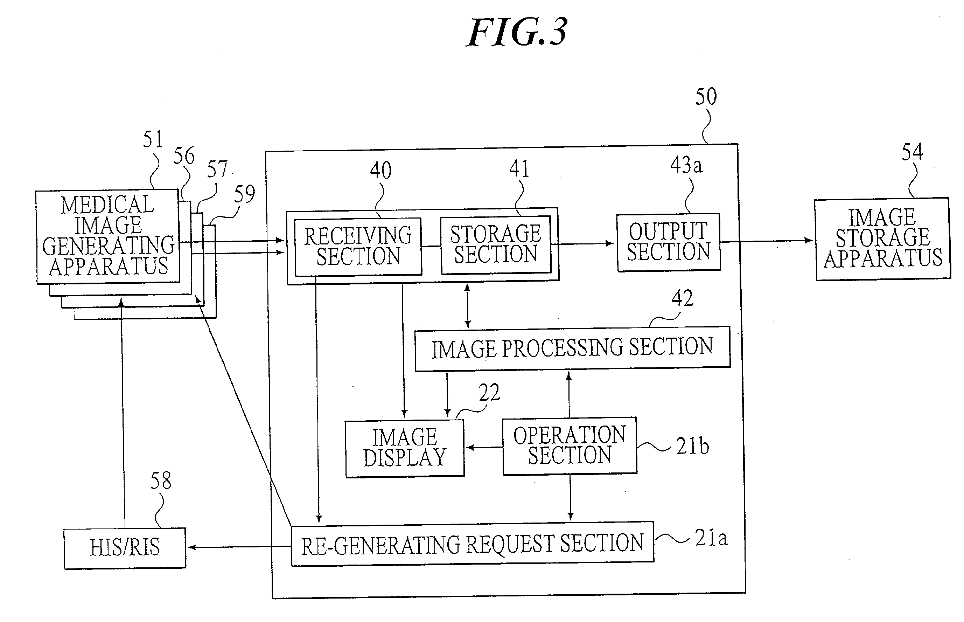

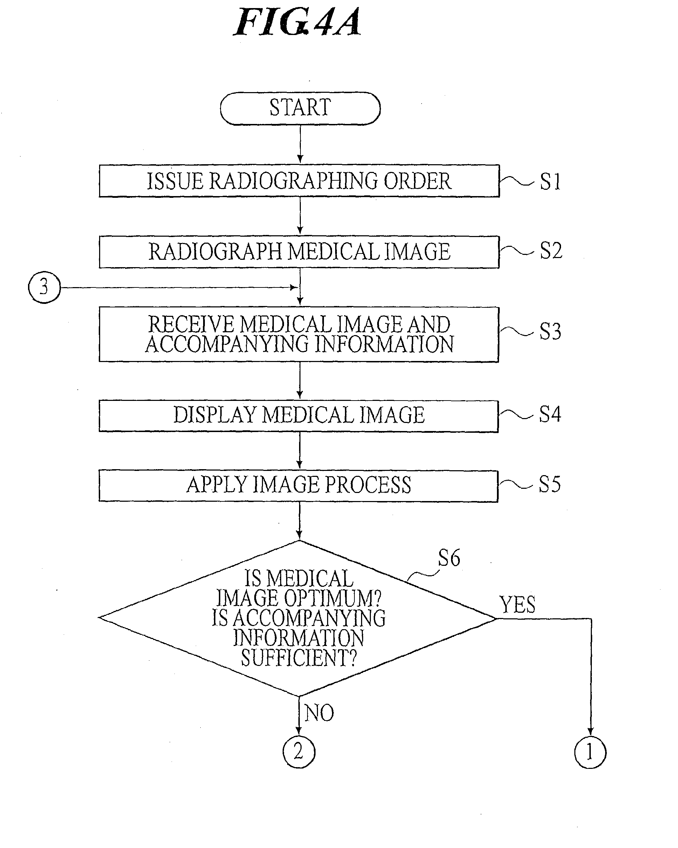

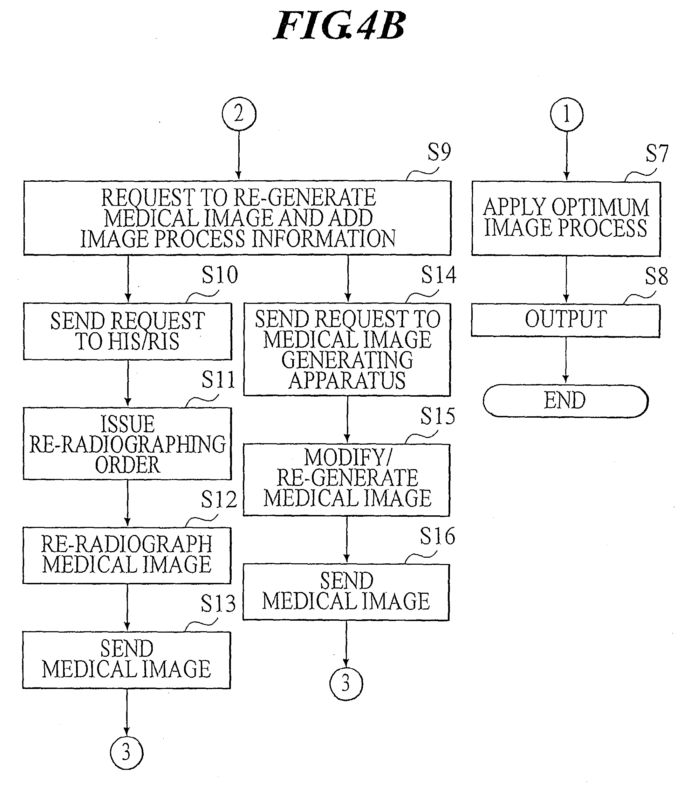

FIG. 3 is a block diagram showing a control system for controlling the image management apparatus 50 shown in FIG. 1 and FIG. 2 to receive images adjusted or corrected by any one of the medical image generating apparatuses 51, 56, 57 and 59. FIG. 4A and FIG. 4B are flow charts for explaining each step of operation of the image management apparatus 50 shown in FIG. 1 and FIG. 2.

As shown in FIG. 3, the image management apparatus 50 shown in FIG. 1 and FIG. 2 comprises a re-generating request section 21a for requesting any one of the medical image generating apparatuses 51, 56, 57, 59 and the HIS/RIS 58 to have any one of the medical image generating apparatuses 51, 56, 57 and 59 re-generate the medical image in a case that it is not possible to apply image processing suitable for at least one of image-forming and image-storing on image information inputted from the storage section 41 for storing the image file received by the receiving section 40 and various types of accompanying information together. Further, the image management apparatus 50 can receive image processing information as additional information to have any one of the medical image generating apparatuses 51, 56, 57 and 59 re-generate a medical image through the operation section 21b and send the image processing information along with request information to have any one of the medical image generating apparatuses 51, 56, 57 and 59 re-generate the medical image from the re-generating request section 21a to any one of the medical image generating apparatuses 51, 56, 57, 59 and the HIS/RIS 58.

Next, operation of the image management apparatus 50 shown in FIG. 1, FIG. 2 and FIG. 3 will be explained with reference to flow charts in FIG. 4A and FIG. 4B.

First, the HIS/RIS 58 issues a radiographing order according to instruction of a doctor or the like to any one of the medical image generating apparatuses 51, 56, 57 and 59 (S1). Based on the radiographing order, for example, the medical image generating apparatus 51 radiographs a medical image and performs necessary image processing (S2). Then, the medical image generating apparatus 51 sends the medical image to the image management apparatus 50 (S3) and the image management apparatus 50 displays the received medical image along with accompanying information thereof on the screen of the image display 22 (S4). Then, the image management apparatus 50 applies image processing on the displayed medical image according to user operation (S5).

Next, when the image management apparatus 50 determines that optimum medical image and sufficient accompanying information are obtained with the above-mentioned image processing and are suitable to be stored in the image storage apparatus 54 (S6;YES), the image management apparatus 50 applies optimum image processing on the optimum medical image (S7) and outputs the image-processed medical image from an output section 43a (FIG. 3) of the output image forming section 43 to the image storage apparatus 54 (S8).

Further, when the image management apparatus 50 does not obtain optimum medical image or sufficient accompanying information (S6;NO), the image management apparatus 50 requests any one of the HIS/RIS 58 and the medical image generating apparatus 51 to have the medical image generating apparatus 51 re-generate the image information (S9). In this case, the image management apparatus 50 adds image processing information for re-generating or re-radiographing so as to obtain an optimum medical image and sufficient accompanying information, and modifies the image processing information that already has been obtained as necessary.

Here, in order to determine whether or not sufficient accompanying information is obtained, a user confirms that the accompanying information is sufficient on the screen. In case the accompanying information can be corrected after the image management apparatus 50 corrects the accompanying information according to operation at the operation section 21b, the user confirms whether or not the accompanying information is sufficient.

Further, DICOM, HL7 or the like, which is a medical image standard network protocol, is used for communication to request the HIS/RIS 58 to have any one of the medical image generating apparatuses 51, 56, 57 and 59 re-generate the image information.

Further, in a case that the medical image processed in Step S6 as mentioned above is a processed medical image, the image management apparatus 50 may request the medical image generating apparatus 51 by sending additional information for instructing to send a combination of an unprocessed medical image and contents of image processing performed by the medical image generating apparatus 51 in Step S9, instead of instructing to re-generate the medical image. In this case, the image management apparatus 50 applies image processing on the unprocessed medical image in comparison with the contents of image processing performed by the medical image generating apparatus 51.

Further, the medical image in the image management apparatus 50 may be either deleted or stored for awhile to be deleted later after the request to re-generate the medical image.

Next, in Step S9 as mentioned above, in a case that the image management apparatus 50 sends a request to the HIS/RIS 58 to have any one of the medical image generating apparatuses 51, 56, 57 and 59 re-generate the medical image (S10), based on the request of re-generation, the HIS/RIS 58 automatically generates a re-radiographing order and issues it to the medical image generating apparatus 51 (S11). Based on the re-radiographing order, the medical image generating apparatus 51 radiographs a medical image and performs necessary image processing (S12). Then, the medical image generating apparatus 51 sends the medical image along with accompanying information thereof to the image management apparatus 50 (S13).

Further, in a case that the image management apparatus 50 request the medical image generating apparatus 51 to re-generate the medical image in Step S9 as mentioned above and sends the request to the medical image generating apparatus 51 (S14), the medical image generating apparatus 51 corrects the medical image such as re-performs image processing or the like (S15) and sends the corrected medical image to the image management apparatus 50 (S16).

Next, in a case that the image management apparatus 50 obtains optimum medical image and sufficient accompanying information at last by returning to Step S3 as mentioned above in which the image management apparatus 50 receives the medical image along with accompanying information thereof sent in Step S13 or S16 as mentioned above and then performing the steps following Step S3 (S6), the image management apparatus 50 applies optimum image processing on the optimum medical image (S7) and outputs the image-processed medical image to the image storage apparatus 54 (S8).

Here, in Step S9 as mentioned above, the request to re-generate may always be sent to the HIS/RIS 58 in order for any one of the medical image generating apparatuses 51, 56, 57 and 59 to correct or re-generate the medical image according to need.

Further, in Step S8, the medical image may be outputted to the image forming apparatus 55. In this case, in order to determine whether or not optimum medical image and sufficient accompanying information are obtained, the image management apparatus 50 confirms whether or not the medical image and accompanying information thereof are suitable for image-forming.

As described above, according to the embodiment of the present invention in FIG. 4A and FIG. 4B, in a case that when performing image processing on a received medical image, the image management apparatus 50 cannot obtain a medical image suitable for at least one of image-forming and image-storing, the image management apparatus 50 can automatically request any one of the medical image generating apparatuses 51, 56, 57, 59 and the HIS/RIS 58 to have any one of the medical image generating apparatuses 51, 56, 57 and 59 re-generate and re-send the medical image. Therefore, the image management apparatus 50 can automatically receive image information such as a medical image re-generated with correction or adjustment by re-radiographing, accompanying information thereof or the like from any one of the medical image generating apparatuses 51, 56, 57 and 59. As a result, the image management apparatus 50 can obtain the image information having a quality medical image and sufficient accompanying information and output the image information suitable for image-forming or image-storing to the image forming apparatus 55 or the image storage apparatus 54. Further, conveniently, it is possible that the image management apparatus 50 automatically receives re-generated image information without having a user travel to the HIS/RIS 58 that is located externally.

Here, the storage section 41 of the image management apparatus 50 shown in FIG. 2 and FIG. 3 stores programs for executing each step in the flow charts in FIG. 4A and FIG. 4B as mentioned above, and the main controller 21 can load and execute the programs according to need.

The present invention has been explained according to the embodiment as above. However, the present invention is not limited to the above-explained embodiment, and can take various types of changes without departing from essence of the present invention. For example, one of the medical image generating apparatuses 51, 56, 57 and 59 may be a modality other than the above-mentioned CR and CT. For example, it may be a medical image generating apparatus for any modality of MRI (Magnetic Resonance Imaging), DR (Digital Radiography), US (Ultrasound) and so on, of course. Further, each of these medical image generating apparatuses may be connected to the medical network system of the present invention.

Further, a protocol such as PPS, Detach or the like may be used for communication for requesting the HIS/RIS 58 to have any one of the medical image generating apparatus 51, 56, 57 and 59 re-generate the medical image.

Further, in FIG. 4A and FIG. 4B, the case that optimum medical image and sufficient accompanying information are obtained with image processing applied by the image management apparatus 50 and judged suitable to be stored in the image storage apparatus 54 (S6) has been explained. However, there is a case that any one of the medical image generating apparatuses 51, 56, 57, 59, the image forming apparatus 55 and the image storage apparatus 54 performs image processing according to user operation. In this case, even when an optimum medical image cannot be obtained with the image processing, it is possible that the image management apparatus 50 regards it as not suitable in Step S6 and requests to have any one of the medical image generating apparatuses 51, 56, 57 and 59 re-generate the medical image.

The entire disclosure of Japanese Patent Application No. Tokugan 2002-266221 filed on September 12, 2002 including specification, claims, drawings and summary are incorporated herein by reference in its entirety.

## Claims

1. An image management apparatus capable of receiving a medical image from a medical image generating apparatus according to a radiographing order issued by an outer information management apparatus, image-processing the medical image received, displaying the medical image image-processed along with accompanying information thereof and outputting the medical image and the accompanying information to at least one of an image forming apparatus and an image storage apparatus, the image management apparatus comprising:
a request section for requesting at least one of the medical image generating apparatus and the outer information management apparatus to have the medical image generating apparatus at least one of re-generate and re-send the medical image, in a case of being impossible to apply image processing suitable for at least one of image-forming in the image forming apparatus and image-storing in the image storage apparatus on the medical image received.

2. The apparatus of claim 1, wherein the request section sends additional information so as to have the medical image generating apparatus re-generate the medical image when requesting at least one of the medical image generating apparatus and the outer information management apparatus to have the medical image generating apparatus re-generate the medical image.

3. The apparatus of any one of claims 1 and 2, wherein the case of being impossible to apply image processing includes at least one of a case that a medical image image-processed by at least one of the medical image generating apparatus, the image management apparatus and the image forming apparatus is not optimum and a case that the accompanying information is insufficient.

4. The apparatus of any one of claims 1 to 3, wherein a medical standard network protocol is used for communication for requesting to have the medical image generating apparatus re-generate the medical image.

5. The apparatus of any one of claims 1 to 4, wherein the additional information so as to have the medical image generating apparatus re-generate the medical image includes information for instructing the medical image generating apparatus to send any one of the medical image which already has been processed, and a combination of the medical image which has not been processed and contents of the image processing performed by the medical image generating apparatus.

6. A medical network system comprising:
an image management apparatus,
a medical image generating apparatus for sending a medical image to the image management apparatus,
at least one of an image storage apparatus for storing the medical image outputted from the image management apparatus and an image forming apparatus for forming the medical image outputted in a recording medium therein, and
an information management apparatus for issuing a radiographing order to the medical image generating apparatus and managing information,
wherein the image management apparatus comprises a request section for requesting at least one of the medical image generating apparatus and the information management apparatus to have the medical image generating apparatus at least one of re-generate and re-send the medical image, in a case of being impossible to apply image processing suitable for at least one of image-forming in the image forming apparatus and image-storing in the image storage apparatus on the medical image received.

7. The system of claim 6, wherein the request section sends additional information so as to have the medical image generating apparatus re-generate the medical image when requesting at least one of the medical image generating apparatus and the outer information management apparatus to have the medical image generating apparatus re-generate the medical image.

8. The system of any one of claims 6 and 7, wherein the case of being impossible to apply image processing includes at least one of a case that a medical image image-processed by at least one of the medical image generating apparatus, the image management apparatus and the image forming apparatus is not optimum and a case that the accompanying information is insufficient.

9. The system of any one of claims 6 to 8, wherein a medical standard network protocol is used for communication for requesting to have the medical image generating apparatus re-generate the medical image.

10. The system of any one of claims 6 to 9, wherein the additional information so as to have the medical image generating apparatus re-generate the medical image includes information for instructing the medical image generating apparatus to send any one of the medical image which already has been processed, and a combination of the medical image which has not been processed and contents of the image processing by the medical image generating apparatus.
